(19) Europäisches Patentamt
European Patent Office
Office européen des brevets

(11) Veröffentlichungsnummer: **0 008 323**
**B1**

# (12) EUROPÄISCHE PATENTSCHRIFT

(45) Veröffentlichungstag der Patentschrift: **25.08.82**

(51) Int. Cl.³: **C 07 D 213/53**, C 07 D 213/48

(21) Anmeldenummer: **79101772.6**

(22) Anmeldetag: **05.06.79**

(54) **Verfahren zur Herstellung von Pyridinaldehyden, Derivaten davon und nach dem Verfahren hergestellte Verbindungen.**

(30) Priorität: **08.06.78 CH 6287/78**

(43) Veröffentlichungstag der Anmeldung: **05.03.80 Patentblatt 80/5**

(45) Bekanntmachung des Hinweises auf die Patenterteilung: **25.08.82 Patentblatt 82/34**

(84) Benannte Vertragsstaaten: **CH DE FR GB IT**

(56) Entgegenhaltungen:
**DE-B-215 156**
**DE-B-1 220 423**
**P. KARLSON »Kurzes Lehrbuch der Biochemie«, 9. Auflage, 1974, GEORG THIEME VERLAG, Stuttgart, Seite 143, Formelschema**

(73) Patentinhaber: **CIBA-GEIGY AG, Patentabteilung Postfach, CH-4002 Basel (CH)**

(72) Erfinder: **Bader, Rolf, Mühlestiegrain 7, CH-4125 Riehen (CH)**
Erfinder: **Reinehr, Dieter, Dr., Wolfsheule 10, D-7842 Kandern (DE)**

(74) Vertreter: **Zumstein, Fritz sen., Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von Pyridinaldehyden, Derivaten davon und nach dem Verfahren hergestellte Verbindungen

Die vorliegende Erfindung betrifft ein neues Verfahren zur Herstellung von Pyridinaldehyden und Derivaten davon.

Aus der Literatur sind zahlreiche Verfahren zur Herstellung von Pyridinaldehyden bekannt. Als nächstvergleichbares Verfahren ist die sogenannte Sommelet-Reaktion zu erwähnen, nach der 3-Pyridinaldehyd durch Umsetzen von 3-Aminomethylpyridin mit Hexamethylentetramin bei einem pH zwischen 3 und 6,5 in einer Ausbeute von 57% d. Th. hergestellt werden kann. Der 2- und 4-Pyridinaldehyd lassen sich jedoch nach dieser Methode nicht herstellen [vgl. J. Chem. Soc., 1953, 1740–41].

Pyridinaldehyde können auch durch katalytische Hydrierung von Cyanpyridinen hergestellt werden; vgl. zum Beispiel deutsche Patentschrift 1 088 958, DDR-Patentschrift 43 044 und US-Patentschriften 3 274 206 und 3 160 633. Diese Hydrierungsverfahren sind insofern unbefriedigend, als sie entweder spezielle Maßnahmen zur Kontrolle der Wasserstoffaufnahme und damit zur Verringerung unerwünschter Nebenreaktionen und/oder hohe Mengen an teuren Katalysatoren erfordern, die zudem teilweise im Reaktionsmedium löslich sind und deren Aufarbeitung aufwendig ist. Ferner muß dabei unter rigorosen Reaktionsbedingungen, wie stark saures Medium, hoher Druck oder hohe Temperaturen, gearbeitet werden. Die Ausbeuten sind zum Teil auch unbefriedigend.

Ebenfalls sehr rigorose Reaktionsbedingungen erfordert die Herstellung von Pyridinaldehyden mittels katalytischer Oxidation von Methylpyridinen in der Gasphase bei Temperaturen zwischen etwa 370 und 420°C. Dieses Verfahren ist sowohl in bezug auf die benötigten Apparaturen als auch hinsichtlich der Reaktionsbedingungen aufwendig (US-Patentschrift 2 749 351).

Gemäß einem weiteren Verfahren (US-Patentschrift 3 008 963) können Pyridinaldehyde auch durch Erhitzen der entsprechenden Pyridin-N-oxid-carbinole auf Temperaturen zwischen etwa 250 und 450°C hergestellt werden. Die Herstellung der Pyridin-N-oxid-carbinole und deren Überführung in die Pyridinaldehyde ist jedoch nicht ungefährlich, da es beim Erhitzen der Pyridin-N-oxid-carbinole zu Explosionen kommen kann. Zudem führt dieses Verfahren nur im Falle des 2-Pyridinaldehyds zu befriedigenden Ausbeuten.

Gegenstand der Erfindung ist daher ein neues Verfahren, mit dem Pyridinaldehyde und Derivate davon auf einfache Weise unter milden, umweltfreundlichen Reaktionsbedingungen in guten bis sehr guten Ausbeuten auch in großtechnischem Maßstab hergestellt werden können.

Nach dem erfindungsgemäßen Verfahren können Verbindungen der Formel I

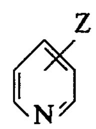

(I)

worin

Z —CHO oder 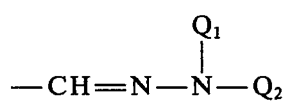

$Q_1$ Wasserstoff oder Alkyl mit 1–4 C-Atomen und

$Q_2$ Phenyl, das durch Halogenatome, Alkyl- oder Alkoxygruppen mit je 1–4 C-Atomen substituiert sein kann, oder Naphthyl bedeuten,

nach einem Eintopfverfahren oder einem Mehrstufenverfahren dadurch hergestellt werden, daß man ein Aminomethylpyridin mit einer mindestens stöchiometrischen Menge einer Verbindung der Formel II

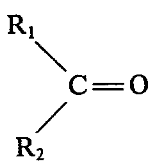

(II)

zu einer Verbindung der Formel III

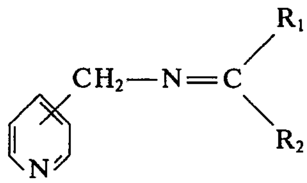

(III)

umsetzt, die Verbindung der Formel III in Gegenwart eines Katalysators der Formel IV

$$(X)^{n\oplus}(OY)^{\ominus}_{n} \qquad (IV)$$

in einem inerten organischen Lösungsmittel zu einer Verbindung der Formel V

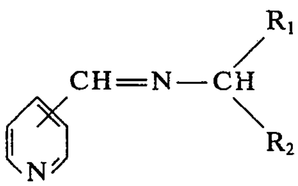

(V)

isomerisiert, wobei in den Formeln II bis V

$R_1$ Wasserstoff oder Alkyl mit 1–8 C-Atomen,
$R_2$ Alkyl mit 1–8 C-Atomen oder
$R_1$ und $R_2$ zusammen Alkylen mit 4–8 C-Atomen,
X ein Alkalimetall- oder Erdalkalimetallion oder die Ammonium-Gruppe

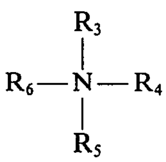

worin $R_3$ bis $R_6$ unabhängig voneinander unsubstituiertes, unverzweigtes oder verzweigtes Alkyl ($C_1-C_{20}$) oder durch Phenyl oder Naphthyl substituiertes Alkyl ($C_1-C_{20}$) bedeuten, insbesondere bedeutet $R_3$, $R_4$ und $R_5$ entweder $CH_3$ oder $C_2H_5$ und $R_6$ eine Alkylgruppe ($C_{10}-C_{20}$),
Y Wasserstoff oder Alkyl mit 1–12 C-Atomen und
n die Ladung des Alkalimetall- oder Erdalkalimetallions

bedeuten und die Verbindung der Formel V in eine Verbindung der Formel I, worin Z –CHO darstellt, überführt, indem man die Verbindung der Formel V mit einer anorganischen Säure behandelt, oder die Verbindung der Formel V in eine Verbindung der Formel I, worin Z die

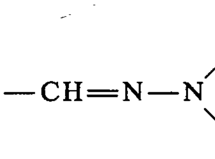 Gruppe

darstellt, überführt, indem man die Verbindung der Formel V in Gegenwart einer Säure mit einer Verbindung der Formel VI

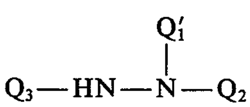

(VI)

umsetzt, worin $Q_2$ die unter Formel I angegebene Bedeutung hat,

$Q_1'$ Wasserstoff, Alkyl mit 1–4 C-Atomen oder $-SO_3^{\ominus}M^{\oplus}$,
$Q_3$ Wasserstoff oder $-SO_3^{\ominus}M^{\oplus}$ und
$M^{\oplus}$ ein Alkalimetallkation, besonders das Natrium- oder Kaliumkation, darstellen.

Alkylgruppen $R_1$ und $R_2$ können geradkettig oder verzweigt sein und weisen bevorzugt 1–4 C-Atome auf. Stellen $R_1$ und $R_2$ beide Alkylgruppen, besonders solche mit 1–4 C-Atomen, dar, so sind diese mit Vorteil geradkettig. Beispiele definitionsgemäßer Alkylgruppen $R_1$ und $R_2$ sind: die Methyl-, Äthyl-, n-Propyl-, Isopropyl-, n-Butyl-, sek.-Butyl-, tert.-Butyl-, n-Pentyl-, n-Hexyl-, n-Heptyl- und n-Octylgruppe.

Bilden $R_1$ und $R_2$ zusammen eine Alkylenkette, so handelt es sich bevorzugt um Tetramethylen und insbesondere um Pentamethylen.

Bevorzugt verwendet man im erfindungsgemäßen Verfahren eine Verbindung der Formel II, worin $R_1$ und $R_2$ je Äthyl oder zusammen Pentamethylen darstellen oder worin $R_1$ Wasserstoff und $R_2$ Isopropyl bedeuten.

Als Aminomethylpyridin verwendet man bevorzugt 3-Aminomethylpyridin und insbesondere 4-Aminomethylpyridin.

Alkylgruppen Y können ebenfalls geradkettig oder verzweigt sein und weisen bevorzugt 1–6 und insbesondere 1–4 C-Atome auf. Besonders bevorzugt stellt Y tert.-Butyl dar. X stellt z. B. Lithium, Kalium, Natrium, Magnesium, Calcium oder Barium dar. Bevorzugt bedeutet X ein Alkalimetall, insbesondere Natrium oder Kalium. Besonders bevorzugte Verbindungen der Formel IV sind das Natrium- oder Kaliummethylat, -äthylat, -isopropylat und vor allem das Natrium- oder Kalium-tert.-butylat. Alkylgruppen $Q_1$ und Alkyl- oder Alkoxysubstituenten an Phenylgruppen $Q_2$ sind mit Vorteil geradkettig und weisen insbesondere 1 oder 2 C-Atome auf. $Q_1$ stellt besonders bevorzugt Wasserstoff oder Methyl dar. Sind Phenylgruppen $Q_2$ durch Halogenatome substituiert, so handelt es sich z. B. um Brom- und vor allem um Chloratome. Phenylgruppen $Q_2$ können mehrere gleiche oder verschiedene definitionsgemäße Substituenten aufweisen, tragen jedoch bevorzugt nur einen der genannten Substituenten. Als geeignete Hydrazine der Formel VI sind beispielsweise zu erwähnen:

Phenylhydrazin, α- oder β-Naphthylhydrazin, Phenyl-N-methylhydrazin, N-Phenyl-N-äthylhydrazin, N-4-Methyl-, N-4-Äthyl-, N-4-Methoxy-, N-4-Äthoxy- und N-4-Chlorphenylhydrazin oder -N-methylhydrazin und die entsprechenden Sulfonsäuresalze. Besonders bevorzugt als Verbindungen der Formel VI sind das N-Phenyl-N-methylhydrazin, das Natriumsalz des 1-(4'-Methoxyphenyl)-2-sulfohydrazins bzw. des 1-(4'-Methoxyphenyl)-1,2-disulfohydrazins und insbesondere das Phenylhydrazin.

Die Aminomethylpyridine sowie die Verbindungen der Formel IV und VI sind an sich bekannt.

Die Umsetzung des Aminomethylpyridins mit einer Verbindung (Aldehyd oder Keton) der Formel II und die Isomerisierung einer Verbindung der Formel III zu einer Verbindung der Formel V werden mit Vorteil in Gegenwart eines inerten organischen Lösungsmittels vorgenommen.

Als inerte organische Lösungsmittel verwendet man zweckmäßig aprotische organische Lösungsmittel, vor allem aliphatische oder aromatische Kohlenwasserstoffe, aliphatische oder cyclische Äther, Äthylenglykol- und Diäthylenglykoldialkyläther, Dialkylsulfoxide mit je 1–4 C-Atomen in den Alkylteilen oder N,N-Dialkylamide von aliphatischen Monocarbonsäuren mit 1–3 C-Atomen im Säureteil und Alkohole. Beispiele solcher Lösungsmittel sind: n-Pentan, n-Hexan, n-Heptan, Benzol, Toluol, Xylole, Diäthyläther, Di-n-propyläther, Tetrahydrofuran, Tetrahydropyran, Dioxan, Äthylenglykol- und Diäthylenglykoldimethyläther und -diäthyläther, Dimethylsulfoxid, N,N-Dimethylformamid, Methanol, Äthanol, n- und iso-Propanol, n-Butanol, tert.-Butanol oder n-Hexanol. Es können auch Gemische derartiger Lösungsmittel verwendet werden. Bevorzugte Lösungsmittel sind Benzol, Dioxan, Tetrahydrofuran, Diäthyläther und insbesondere Toluol.

Die Reaktionstemperaturen bei der Isomerisierung, d. h., die Überführung der Verbindung III in die Verbindung V, liegen beim erfindungsgemäßen Verfahren zweckmäßig zwischen 0 und 80°C und besonders zwischen etwa 10 und 60°C.

Die Verbindung der Formel II wird in mindestens stöchiometrischer Menge, bezogen auf das Aminomethylpyridin, eingesetzt.

Mit Vorteil verwendet man einen leichten Überschuß an Verbindung der Formel II, z. B. einen etwa 5–20%igen Überschuß.

Die Katalysatoren der Formel IV verwendet man zweckmäßig in einer Menge von mindestens 0,1 Mol-%, bezogen auf die Verbindung der Formel III. Bevorzugt sind Mengen von etwa 0,5 bis 15 Mol-%, bezogen auf die Verbindung der Formel III. Die Überführung von Verbindungen der Formel V in Verbindungen der Formel I mit Z = –CHO erfolgt bevorzugt in stark saurem wäßrigem Medium. Als Säure verwendet man zweckmäßig eine anorganische Säure, wie Salzsäure oder Schwefelsäure. Die Säure wird im allgemeinen in mindestens stöchiometrischer Menge, bezogen auf die Verbindung der Formel V, und bevorzugt im Überschuß eingesetzt. Erfindungsgemäß erhaltene Pyridinaldehyde der Formel I können gewünschtenfalls auch auf an sich bekannte Weise durch Umsetzung mit Hydrazinen der Formel VI in die entsprechenden Hydrazone der Formel I (vgl. zum Beispiel deutsche Patentschrift 1 133 054) oder auch in andere Derivate, wie Aldoxime, Semicarbazone oder Thiosemicarbazone, übergeführt werden. Definitionsgemäße Hydrazone der Formel I können jedoch nach dem erfindungsgemäßen Verfahren auch ohne Zwischenisolierung der Pyridinaldehyde unter milden Reaktionsbedingungen direkt aus den Verbindungen der Formel V hergestellt werden. Dies ist im Hinblick auf die Oxidationsempfindlichkeit der Pyridinaldehyde und/oder die Weiterverwendung der Verbindungen der Formel I mit Z ≠ –CHO, beispielsweise zur Herstellung von kationischen Farbstoffen, von besonderem Vorteil.

Die direkte Umsetzung der Verbindungen der Formel V mit den Hydrazinen der Formel VI zu Verbindungen der Formel I mit

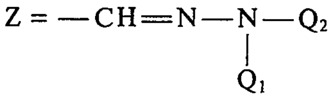

wird je nach Art des Hydrazins in wäßrigem oder organischem Medium und in Gegenwart einer anorganischen oder organischen Säure vorgenommen. Stellen $Q_3$ und/oder $Q_1'$ eine $-SO_3^{\ominus}M^{\oplus}$-Gruppe dar, so führt man die Umsetzung vorzugsweise in wäßrigem Medium und in Gegenwart einer anorganischen Säure, wie Salzsäure, durch. Dabei wird die Säure zweckmäßig in mindestens stöchiometrischer Menge eingesetzt. Bedeuten $Q_3$ Wasserstoff und $Q_1'$ Wasserstoff oder Alkyl, so erfolgt die Umsetzung zweckmäßig in Gegenwart eines inerten organischen Lösungsmittels und einer organischen Säure. In diesem letzteren Fall werden im allgemeinen nur katalytische Mengen Säure benötigt. Als organische Lösungsmittel verwendet man dabei zweckmäßig inerte aprotische organische Lösungsmittel der vorerwähnten Art oder Gemische davon. Als organische Säure setzt man mit Vorteil aliphatische oder aromatische Monocarbonsäuren oder Monosulfonsäuren ein, wie Ameisensäure, Essigsäure, Propionsäure, Buttersäure, n-Valeriansäure, Methansulfonsäure, Benzoesäure, Benzolsulfonsäure und p-Toluolsolfonsäure. Besonders bevorzugt ist wasserfreie Essigsäure.

Die bei der Überführung in Verbindungen der Formel I als Nebenprodukte entstehenden Amine

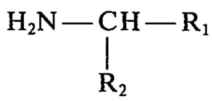

können leicht aus dem Reaktionsmedium abgetrennt und wieder verwertet werden.

Die Zwischenprodukte der Formel III und/oder V können gewünschtenfalls auf an sich bekannte Weise isoliert werden, beispielsweise mittels Destillation. Eine solche Zwischenisolierung ist jedoch nicht unbedingt erforderlich, und ein besonderer Vorteil des erfindungsgemäßen Verfahrens besteht darin, daß dieses ohne nennenswerte Verringerung der Ausbeute an Verbindung der Formel I auch im Eintopfverfahren durchgeführt werden kann.

Nach Beendigung der Umsetzung können die Verbindungen der Formel I auf übliche Weise isoliert und gereinigt werden, z. B. bei $Z = -CHO$ durch Extrahieren mit einem geeigneten organischen Lösungsmittel. Die Verbindungen der Formel I, worin Z

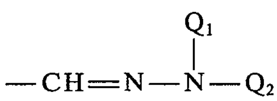

darstellt, fallen im allgemeinen in kristalliner Form an. Die Verbindungen der Formel I lassen sich nach dem erfindungsgemäßen Verfahren in sehr guten Ausbeuten (ca. 70–95, insbesondere 80% d. Th., bezogen auf das Ausgangs-Aminomethylpyridin) herstellen.

Die bevorzugte Verfahrensvariante besteht darin, daß man, ausgehend von Aminomethylpyridin, im Eintopfverfahren Pyridinverbindungen der Formel I herstellt, worin Z die

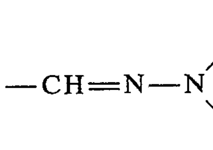 Gruppe

darstellt, d. h., man setzt die Verbindung VI (Hydrazin) ein.

Die Verbindungen der Formel I sind bekannt und stellen wertvolle Zwischenprodukte (z. B. das 2- oder insbesondere 4-Pyridinhydrazon) zur Herstellung verschiedener Derivate, wie Arzneimittel, Herbizide oder Farbstoffe, vor allem kationische Farbstoffe, dar (vgl. zum Beispiel US-Patentschriften 2 749 351, 3 160 633 und 3 274 206 sowie deutsche Patentschrift 1 133 054).

Die folgenden Beispiele veranschaulichen die Arbeitsweise des erfindungsgemäßen Verfahrens. Pa bedeutet Pascal = internationale Druckeinheit.

## Beispiel 1

### a) Herstellung von Isobutyliden-4-aminomethylpyridin

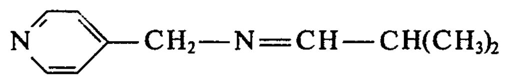

227 g (3,15 Mol) Isobutyraldehyd werden zu einer Lösung von 324 g (3,0 Mol) 4-Aminomethylpyridin in 300 ml Benzol innerhalb von 1,5 Stunden so zugetropft, daß die Temperatur in der Lösung 35° C nicht übersteigt. Man trennt das entstandene Wasser (ca. 39 g) ab und destilliert das Benzol (mit Restwasser) bei leichtem Wasserstrahlvakuum ab. Der Rückstand wird im Ölpumpenvakuum destilliert. Nach einer kleinen Vorfraktion von 5 g erhält man 445 g (2,75 Mol) Isobutyliden-4-aminomethylpyridin als leicht gelbliche Hauptfraktion, entsprechend einer Ausbeute von 91,5% d. Th.; Sdp. 81,5 – 82° C/133 Pa; $n_D^{20}$ = 1,5105.

Analyse für $C_{10}H_{14}N_2$ (Molgewicht 162,24):

| | | | |
|---|---|---|---|
| Berechnet | C 74,03, | H 8,70, | N 17,27%, |
| gefunden | C 73,9, | H 8,8, | N 17,5%. |

$^1$H-NMR-Spektrum $\tau$ [ppm]: 1,49(d), 2,23(d), 2,83(d), 5,48(s), 7,48(m), 8,87(d) im Verhältnis 2 : 1 : 2 : 2 : 1 : 6.

### b) Herstellung von 4-Pyridylmethyliden-isobutylamin

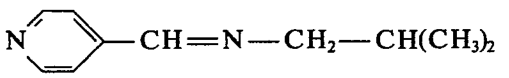

Zu einer Suspension von 2 g (0,0179 Mol) Kalium-tert.-butylat in 100 ml Toluol werden unter Rühren bei 40° C 96 g (0,593 Mol) Isobutyliden-4-aminomethylpyridin innerhalb von 30 Minuten zugetropft. Die Reaktionsmischung wird dabei dunkelrotbraun. Es wird noch 1,5 Stunden bei 40° C gerührt, und das Toluol wird im Wasserstrahlvakuum abdestilliert. Der Rückstand wird dann im Ölpumpenvakuum destilliert. Man erhält 85 g (0,525 Mol) 4-Pyridylmethyliden-isobutylamin als leicht gelbliche Flüssigkeit, entsprechend einer Ausbeute von 88,5% d. Th.; Sdp. 62 – 63° C/1,3 Pa; $n_D^{20}$ = 1,5166.

$^1$H-NMR-Spektrum $\tau$ [ppm]: 1,36(d), 1,82(s), 2,45(d), 6,54(dd), 7,99(m), 9,06(d) im Verhältnis 2 : 1 : 2 : 2 : 1 : 6.

### c) Herstellung von 4-Pyridinaldehyd-phenylhydrazon

100 g (0,617 Mol) 4-Pyridylmethyliden-isobutylamin werden in 100 ml Toluol gelöst, die Lösung wird mit 2 g Eisessig versetzt, und innerhalb von 25 Minuten werden 67,5 g (0,649 Mol) Phenylhydrazin zugetropft. Die Temperatur steigt dabei auf 60° C an. Nach der Zugabe von ca. 2/3 des Phenylhydrazins beginnt das Produkt in Form von gelben Nädelchen auszufallen. Es wird noch 1 Stunde bei Raumtemperatur (20 – 25° C) nachgerührt, dann abfiltriert, mit wenig kaltem Toluol gewaschen und bei 50° C im Vakuumtrockenschrank getrocknet. Man erhält 115 g (0,584 Mol) 4-Pyridinaldehyd-phenylhydrazon als feinkristallines, gelbes Produkt, entsprechend einer Ausbeute von 94,5% d. Th.; Fp. 178 – 180° C. (Ausbeute total 78%, bezogen auf 4-Aminomethylpyridin.)

Analyse für $C_{12}H_{11}N_3$ (Molgewicht 197,24):

| | | | |
|---|---|---|---|
| Berechnet | C 73,08, | H 5,62, | N 21,31%, |
| gefunden | C 73,1, | H 5,6, | N 21,4%. |

d) Überführung von 4-Pyridinaldehyd-phenylhydrazon in einen Farbstoff

19,7 g des nach dem obigen Verfahren hergestellten 4-Pyridinaldehydphenylhydrazons werden in 250 ml Wasser suspendiert. Nach Zugabe von 2,0 g Magnesiumoxyd werden bei 25–30°C unter Rühren 25,2 g Dimethylsulfat zugetropft. Man läßt 2 Stunden nachrühren, filtriert die erhaltene Farbstofflösung, kühlt das Filtrat dann auf 0°C ab und gibt 10,0 g Dimethylsulfat und danach 40 ml 30%ige Natronlauge zu. Man läßt das Reaktionsgemisch 1 Stunde verrühren, wobei die Innentemperatur mittels Eiszugabe unterhalb 20°C gehalten wird. Dann stellt man mittels konzentrierter Salzsäure den pH-Wert auf 4–5 und scheidet den Farbstoff der Formel

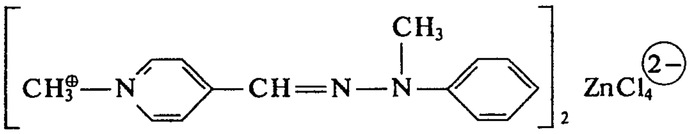

durch tropfenweise Zugabe von 13,0 ml 50%iger Zinkchloridlösung ab.

## Beispiel 2

75 g (1,04 Mol) Isobutyraldehyd werden innerhalb von einer Stunde zu einer Lösung von 102 g (0,945 Mol) 4-Aminomethylpyridin in 350 ml Toluol zugetropft. Nach beendetem Eintropfen wird die entstandene trübe Emulsion noch eine Stunde bei Raumtemperatur gerührt, und das gebildete Wasser wird abgetrennt. Der überschüssige Aldehyd und das Restwasser werden anschließend azeotrop im Wasserstrahlvakuum abgezogen, wobei ungefähr die Hälfte des eingesetzten Toluols mit überdestilliert. Dann wird die toluolische Lösung mit 5 g (0,045 Mol) Kalium-tert-butylat versetzt, wobei die Reaktionsmischung eine dunkelviolette Farbe annimmt, und 1,5 Stunden bei 35°C gerührt. Nach dem Ansäuren mit 5 g Eisessig werden innerhalb von 15 Minuten 107 g (1,03 Mol) Phenylhydrazin zugetropft, wobei das Phenylhydrazon in Form von gelben Nädelchen ausfällt. Man erhält 142,5 g (0,723 Mol) 4-Pyridinaldehydphenylhydrazon, entsprechend einer Ausbeute von 76,5% d. Th., bezogen auf eingesetztes 4-Aminomethylpyridin; Fp. 177–179°C.

## Beispiel 3

Verwendet man im Beispiel 1b) bei sonst gleicher Arbeitsweise anstelle von 100 ml Toluol 100 ml Dioxan, so erhält man nach der weiteren Umsetzung und Aufarbeitung wie im Beispiel 1c) beschrieben das 4-Pyridinaldehyd-phenylhydrazon in einer Ausbeute von 69,5% d. Th., bezogen auf das eingesetzte 4-Aminomethylpyridin.

## Beispiel 4

Verwendet man in Beispiel 1b) anstelle von 100 ml Toluol die gleiche Menge Tetrahydrofuran unter Erniedrigung der Reaktionstemperatur auf 0°C, so erhält man nach der weiteren Umsetzung und Aufarbeitung wie in Beispiel 1c) beschrieben das 4-Pyridinaldehydphenylhydrazon in einer Ausbeute von 70,5% d. Th., bezogen auf das eingesetzte 4-Aminomethylpyridin.

## Beispiel 5

Verwendet man in Beispiel 1b) bei sonst gleicher Arbeitsweise anstelle von 100 ml Toluol die gleiche Menge Diäthyläther, so erhält man nach der weiteren Umsetzung und Aufarbeitung wie in Beispiel 1c) beschrieben das 4-Pyridinaldehyd-phenylhydrazon in einer Ausbeute von 68% d. Th., bezogen auf das eingesetzte 4-Aminomethylpyridin.

## Beispiel 6

a) Herstellung von Isobutyliden-3-aminomethylpyridin

Es wird wie in Beispiel 1a) vorgegangen, jedoch unter Verwendung von 54,07 g (0,5 Mol) 3-Aminomethylpyridin, 40 g (0,55 Mol) Isobutyraldehyd und 75 ml Toluol. Nach der Destillation erhält man 80,1 g (0,495 Mol) Isobutyliden-3-aminomethylpyridin; Ausbeute 98,8% d. Th.; Sdp. 106–108°C/ $1,8 \times 10^3$ Pa; $n_D^{20} = 1,5103$.

Analyse für $C_{10}H_{14}N_2$ (Molgewicht 162,24):

| | | | |
|---|---|---|---|
| Berechnet | C 74,03, | H 8,70, | N 17,27%, |
| gefunden | C 73,44, | H 8,74, | N 16,90%. |

MS-Spektrum: Molekül-Peak 162, Bruchstückmassen 147, 133, 119, 92, 65.

$^1$H-NMR-Spektrum $\tau$ [ppm]: 1,5(m), 2,2-2,5(m), 2,78(dd), 5,44(s), 7,5(m), 8,84(d) im Verhältnis von 2 : 2 : 1 : 2 : 1 : 6.

b) Herstellung von 3-Pyridylmethyliden-isobutylamin

Es wird wie in Beispiel 1b) vorgegangen, jedoch unter Verwendung von 80,1 g (0,495 Mol) Isobutyliden-3-aminomethylpyridin, 2,5 g (0,0223 Mol) Kalium-tert-butylat und 75 ml Toluol. Nach einer Reaktionszeit von 1 Stunde bei 30–35°C und nach der Destillation erhält man 74,5 g (0,46 Mol) 3-Pyridylmethylidenisobutylamin; Ausbeute 93% d. Th.; Sdp. 108° C/2 × $10^3$ Pa; $n_D^{20}$ = 1,5198.

Analyse für $C_{10}H_{14}N_2$ (Molgewicht 162,24):

| | | | |
|---|---|---|---|
| Berechnet | C 74,03, | H 8,70, | N 17,27%, |
| gefunden | C 73,84, | H 8,78, | N 17,18%. |

MS-Spektrum: Molekül-Peak 162, Bruchstückmassen 147, 119, 105, 92.

$^1$H-NMR-Spektrum $\tau$ [ppm]: 1,12(d), 1,36(dd), 1,72(s), 1,88(dt), 2,69(dd), 6,54(dd), 7,97(m), 8,98(d) im Verhältnis 1 : 1 : 1 : 1 : 1 : 2 : 1 : 6.

c) Herstellung von 3-Pyridinaldehyd

81 g (0,5 Mol) 3-Pyridylmethyliden-isobutylamin werden zu einer Lösung von 100 ml 37%iger Salzsäure und 100 ml Wasser zugetropft. Die Lösung wird dann mit wäßriger Natronlauge auf pH 3 gestellt, mit Natriumnitrat gesättigt und mit drei 150 ml Portionen Chloroform extrahiert. Nach der Trocknung der Chloroformphase mit wasserfreiem Natriumsulfat und Abdestillieren des Lösungsmittels erhält man 47,5 g (0,44 Mol) 3-Pyridinaldehyd.

Ausbeute 88% d. Th.; Sdp. 80–82° C/1,7 × $10^3$ Pa; $n_D^{20}$ = 1,5488.

Der erhaltene Pyridinaldehyd kann auf an sich bekannte Weise durch Umsetzung mit geeigneten Hydrazinen und anschließende Quaternierung des erhaltenen Pyridinhydrazons in kationische Farbstoffe übergeführt werden, die auf Polyacrylnitrilfasern Färbungen von sehr guten Echtheitseigenschaften ergeben.

Beispiel 7

Es wird wie in Beispiel 2 beschrieben vorgegangen, jedoch unter Verwendung von 54 g (0,5 Mol) 3-Aminomethylpyridin, 120 ml Toluol, 40 g (0,55 Mol) Isobutyraldehyd, 2,5 g (0,0225 Mol) Kalium-tert-butylat und 54 g (0,5 Mol) Phenylhydrazin. Nach der Aufarbeitung erhält man 78,6 g (0,4 Mol) 3-Pyridinaldehydphenylhydrazon, entsprechend einer Ausbeute von 80% d. Th., bezogen auf eingesetztes 3-Aminomethylpyridin; Fp. 156–157° C.

Analyse für $C_{12}H_{11}N_3$ (Molgewicht 197,24):

| | | | |
|---|---|---|---|
| Berechnet | C 73,08, | H 5,62, | N 21,31%, |
| gefunden | C 72,65, | H 5,59, | N 21,32%. |

MS-Spektrum: Molekül-Peak 197, Bruchstückmassen 169, 92, 65.

### Beispiel 8

#### a) Herstellung von 1-Äthylpropyliden-3-aminomethylpyridin

Es wird wie in Beispiel 1a) beschrieben vorgegangen, jedoch unter Verwendung von 54,1 g (0,5 Mol) 3-Aminomethylpyridin, 45,2 g (0,525 Mol) Diäthylketon und 80 ml Benzol. Nach einer Reaktionszeit von 4 Stunden bei Rückflußtemperatur am Wasserabscheider und anschließender Destillation erhält man 83 g (0,472 Mol) 1-Äthylpropyliden-3-aminomethylpyridin; Ausbeute 94,2% d. Th.; Sdp. 75° C/4 Pa.

Analyse für $C_{11}H_{16}N_2$ (Molgewicht 176,26):

| | | | |
|---|---|---|---|
| Berechnet | C 74,96, | H 9,15, | N 15,89%, |
| gefunden | C 74,80, | H 9,30, | N 15,80%. |

MS-Spektrum: Molekül-Peak 176, Bruchstückmassen 147, 92, 65.

$^1$H-NMR-Spektrum $\tau$ [ppm]: 1,5(m), 2,3(m), 2,8(dd), 5,48(s), 7,65(m), 8,87(m) im Verhältnis 2 : 1 : 1 : 2 : 4 : 6.

#### b) Herstellung von 3-Pyridinaldehydphenylhydrazon

Zu einer Aufschlämmung von 2 g (0,0179 Mol) Kalium-tert-butylat in 80 ml Toluol und 10 ml Dimethylsulfoxid werden 80,39 g (0,45 Mol) 1-Äthylpropyliden-3-aminomethylpyridin gegeben, wobei die Temperatur sofort von 22° C auf 26° C ansteigt. Nach einer Reaktionszeit von 5 Stunden bei 35° C werden innerhalb von 10 Minuten 50 g (0,48 Mol) Phenylhydrazon und 1 g Eisessig zugetropft. Man erhält 68,5 g (0,348 Mol) 3-Pyridinaldehyd-phenylhydrazon; Ausbeute 77,2% d. Th.; Fp. 156° C.

### Beispiel 9

#### a) Herstellung von Cyclohexyliden-3-aminomethylpyridin

Es wird wie in Beispiel 8a) beschrieben vorgegangen, jedoch unter Verwendung von 54,1 g (0,5 Mol) 3-Aminomethylpyridin und 55 g (0,561 Mol) Cyclohexanon.

Nach der Destillation erhält man 89,5 g (0,476 Mol) Cyclohexyliden-3-aminomethylpyridin; Ausbeute 95% d. Th.; Sdp. 107° C/4 Pa.

Analyse für $C_{12}H_{16}N_2$ (Molgewicht 188,27):

| | | | |
|---|---|---|---|
| Berechnet | C 76,56, | H 8,57, | N 14,88%, |
| gefunden | C 75,79, | H 8,72, | N 14,63%. |

MS-Spektrum: Molekül-Peak 188, Bruchstückmassen 173, 159, 145, 92, 80, 65.

$^1$H-NMR-Spektrum $\tau$ [ppm]: 1,5(m), 2,35(m), 2,8(m), 5,49(s), 7,6(m), 8,3(m) im Verhältnis 2 : 1 : 1 : 2 : 4 : 6.

#### b) Herstellung von 3-Pyridinaldehyd-phenylhydrazon

Es wird wie in Beispiel 8b) beschrieben vorgegangen, jedoch unter Verwendung von 87,2 g (0,464 Mol) Cyclohexyliden-3-aminomethylpyridin anstelle von 80,39 g (0,45 Mol) 1-Äthylpropyliden-3-aminomethylpyridin. Man erhält 66,5 g (0,338 Mol) 3-Pyridinaldehyd-phenylhydrazon; Ausbeute 72,8% d. Th.; Fp. 156 – 157° C.

### Beispiel 10

#### a) Herstellung von Isobutyliden-2-aminomethylpyridin

Es wird wie in Beispiel 1a) beschrieben vorgegangen, jedoch unter Verwendung von 216 g (2 Mol) 2-Aminomethylpyridin und 144 g (2 Mol) Isobutyraldehyd. Nach einer Reaktionszeit von 2 Stunden bei 35 – 40° C und anschließender Destillation erhält man 277 g (1,71 Mol) Isobutyliden-2-aminomethylpyridin; Ausbeute 85,5% d. Th.; Sdp. 69° C/200 Pa.

Analyse für $C_{10}H_{14}N_2$ (Molgewicht 162,24):

| | | | |
|---|---|---|---|
| Berechnet | C 74,03, | H 8,70, | N 17,27%, |
| gefunden | C 73,74, | H 8,72, | N 16,88%. |

$^1$H-NMR-Spektrum $\tau$ [ppm]: 1,44(d), 2,2–2,45(m), 2,6–2,95(m), 5,29(s), 7,45(m), 8,83(d) im Verhältnis 1 : 2 : 2 : 2 : 1 : 6.

b) Herstellung von 2-Pyridylmethyliden-isobutylamin

Es wird wie in Beispiel 1b) beschrieben vorgegangen, jedoch unter Verwendung von 138 g (0,85 Mol) Isobutyliden-2-aminomethylpyridin, 150 ml Toluol und 4 g (0,036 Mol) Kalium-tert-butylat. Nach einer Reaktionszeit von 30 Minuten bei 40°C und anschließender Destillation erhält man 92 g (0,57 Mol) 2-Pyridylmethyliden-isobutylamin; Ausbeute 67% d. Th.; Sdp. 95°C/2,0 x $10^3$ Pa.

Analyse für $C_{10}H_{14}N_2$ (Molgewicht 162,24):

| | | | |
|---|---|---|---|
| Berechnet | C 74,03, | H 8,70, | N 17,27%, |
| gefunden | C 74,11, | H 8,69, | N 17,14%. |

MS-Spektrum: Molekül-Peak 162, Bruchstückmassen 147, 119, 92, 65.

$^1$H-NMR-Spektrum $\tau$ [ppm]: 1,35(d), 1,62(s), 1,97(d), 2,28(dt), 2,7(m), 6,48(dd), 7,92(sep), 8,96(d) im Verhältnis 1 : 1 : 1 : 1 : 1 : 2 : 1 : 6.

c) Herstellung von 2-Pyridinaldehyd-phenylhydrazon

Es wird wie in Beispiel 1c) beschrieben vorgegangen, jedoch unter Verwendung von 100 g (0,617 Mol) 2-Pyridylmethylidenisobutylamin. Nach der Zugabe von 67,5 g (0,649 Mol) Phenylhydrazon erhält man 116,5 g (0,592 Mol) 2-Pyridinaldehyd-phenylhydrazon, entsprechend einer Ausbeute von 95,9% d. Th.; Fp. 179°C.

Analyse für $C_{12}H_{11}N_2$ (Molgewicht 197,24):

| | | | |
|---|---|---|---|
| Berechnet | C 73,08, | H 5,62, | N 21,31%, |
| gefunden | C 73,2, | H 5,6, | N 21,4%. |

## Beispiel 11

Es wird wie in Beispiel 1b) beschrieben vorgegangen, jedoch unter Verwendung von 35 ml (0,0175 Mol) einer 0,5molaren toluolischen Lösung von Trimethylalkylammoniumhydroxid $[N(CH_3)_3R]OH$ ($R = C_8 - C_{18}$, durchschnittliches Molgewicht = 266,05) anstelle von 2 g (0,0179 Mol) Kalium-tert.-butylat. Nach einer Reaktionszeit von 15 Stunden bei 40°C erhält man das 4-Pyridylmethyliden-isobutylamin in einer Ausbeute von 40% d. Th.

## Beispiel 12

Es wird wie in Beispiel 2 beschrieben vorgegangen, jedoch unter Verwendung einer Lösung von 1,3 g (0,0116 Mol) Kalium-tert.-butylat in 8 ml tert. Butanol anstelle von 5 g (0,045 Mol) Kalium-tert.-butylat. Nach einer Reaktionszeit von 5 Stunden bei 45°C erhält man das 4-Pyridinaldehydphenylhydrazon in einer Ausbeute von 76% d. Th.

## Beispiel 13

400 ml einer Lösung des Natriumsalzes von p-Methoxyphenylhydrazin-$\alpha,\beta$-disulfonsäure (hergestellt aus 30,75 g (0,25 Mol) p-Anisidin durch Diazotierung und Sulfitreduktion) werden in ein Gemisch von 32,4 g (0,20 Mol) 4-Pyridylmethyliden-isobutylamin in 35 ml Toluol und 250 ml konz. Salzsäure eingetropft. Nach 15minütigem Rühren bei 30°C wurde mit Natronlauge neutralisiert und das ausgefallene 4-Pyridinaldehyd-p-methoxyphenylhydrazon abfiltriert, mit Wasser gewaschen und getrocknet.

Ausbeute: 42 g, entsprechend 92% bezogen auf die Schiffbase. Smp. 147–152°C.

**Patentansprüche**

1. Verfahren zur Herstellung einer Verbindung der Formel I

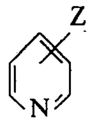

(I)

worin

Z —CHO oder 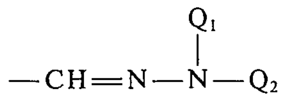

$Q_1$ Wasserstoff oder Alkyl mit 1–4 C-Atomen und

$Q_2$ Phenyl, das durch Halogenatome, Alkyl- oder Alkoxygruppen mit je 1–4 C-Atomen substituiert sein kann, oder Naphthyl bedeuten,

dadurch gekennzeichnet, daß man ein Aminomethylpyridin mit einer mindestens stöchiometrischen Menge einer Verbindung der Formel II

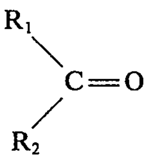

(II)

zu einer Verbindung der Formel III

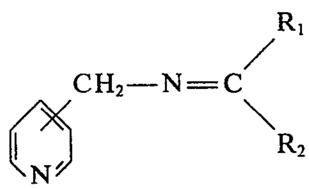

(III)

umsetzt, die Verbindung der Formel III in Gegenwart eines Katalysators der Formel IV

$(X)^{n\oplus}(OY)_n^{\ominus}$ (IV)

in einem inerten organischen Lösungsmittel zu einer Verbindung der Formel V

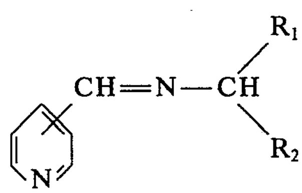

(V)

isomerisiert, wobei in den Formeln II bis V

| | |
|---|---|
| $R_1$ | Wasserstoff oder Alkyl mit 1–8 C-Atomen, |
| $R_2$ | Alkyl mit 1–8 C-Atomen oder |
| $R_1$ und $R_2$ | zusammen Alkylen mit 4–8 C-Atomen, |
| X | ein Alkalimetall- oder Erdalkalimetallion, oder die Ammonium-Gruppe |

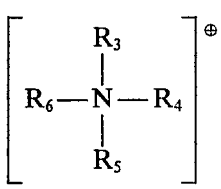

worin $R_3$ bis $R_6$ unabhängig voneinander unsubstituiertes, unverzweigtes oder

verzweigtes Alkyl ($C_1-C_{20}$) oder durch Phenyl oder Naphthyl substituiertes Alkyl ($C_1-C_{20}$) bedeuten,

Y Wasserstoff oder Alkyl mit 1–12 C-Atomen und

n die Ladung des Alkalimetall- oder Erdalkalimetallions bedeuten,

und die Verbindung der Formel V in eine Verbindung der Formel I, worin Z –CHO darstellt, überführt, indem man die Verbindung der Formel V mit einer anorganischen Säure behandelt oder die Verbindung der Formel V in eine Verbindung der Formel I, worin Z die

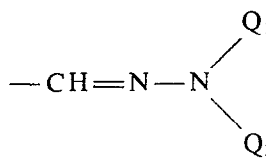 Gruppe

darstellt überführt, indem man die Verbindung der Formel V in Gegenwart einer Säure mit einer Verbindung der Formel VI

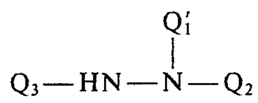 (VI)

umsetzt, worin

$Q_2$ die unter Formel I angegebene Bedeutung hat,

$Q_1'$ Wasserstoff, Alkyl mit 1–4 C-Atomen oder $-SO_3^{\ominus}M^{\oplus}$,

$Q_3$ Wasserstoff oder $-SO_3^{\ominus}M^{\oplus}$ ist und

$M^{\oplus}$ ein Alkalimetallkation darstellt.

2. Verfahren nach Anspruch 1, dadurch gekennzeichnet, daß man die Umsetzung des Aminomethylpyridins mit einer Verbindung der Formel II in Gegenwart eines inerten organischen Lösungsmittels durchführt.

3. Verfahren nach Anspruch 1, dadurch gekennzeichnet, daß man ausgehend von Aminomethylpyridin im Eintopfverfahren die Verbindungen der Formel I herstellt.

4. Verfahren nach Anspruch 1, dadurch gekennzeichnet, daß man die Isomerisierung der Verbindung III bei einer Temperatur zwischen 0 und 80° C und besonders 10 und 60° C vornimmt.

5. Verfahren nach Anspruch 1, gekennzeichnet durch die Verwendung von 3-Aminomethylpyridin und insbesondere 4-Aminomethylpyridin.

6. Verfahren nach Anspruch 1, dadurch gekennzeichnet, daß man eine Verbindung der Formel II verwendet, worin $R_1$ und $R_2$ je Äthyl oder zusammen Pentamethylen darstellen oder worin $R_1$ Wasserstoff und $R_2$ Isopropyl bedeuten.

7. Verfahren nach Anspruch 1, dadurch gekennzeichnet, daß man eine Verbindung der Formel IV verwendet, worin X ein Alkalimetallion, besonders das Natrium- oder Kaliumion, und Y Alkyl mit 1–4 C-Atomen, besonders tert-Butyl, darstellen.

8. Verfahren nach Anspruch 1 zur Herstellung einer Verbindung der Formel I, worin Z

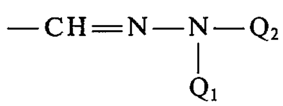

darstellt und $Q_1$ und $Q_2$ die unter Formel I angegebene Bedeutung haben, dadurch gekennzeichnet, daß man die Umsetzung einer Verbindung der Formel V mit einer Verbindung der Formel VI, worin $Q_3$ Wasserstoff und $Q_1'$ Wasserstoff oder Alkyl mit 1–4 C-Atomen bedeuten, in Gegenwart eines inerten organischen Lösungsmittels und einer organischen Säure und die Umsetzung mit einer Verbindung der Formel VI, worin $Q_3$ und/oder $Q_1'$ eine $-SO_3^{\ominus}M^{\oplus}$-Gruppe bedeuten, in wässerigem Medium und in Gegenwart einer anorganischen Säure vornimmt.

9. Verfahren nach Anspruch 8, dadurch gekennzeichnet, daß man als organische Säure eine aliphatische oder aromatische Monocarbonsäure oder Monosulfonsäure verwendet.

10. Verfahren nach Anspruch 8, dadurch gekennzeichnet, daß man als Verbindung der Formel VI N-Phenyl-N-methylhydrazin, das Natriumsalz des 1-(4'-Methoxyphenyl)-2-sulfohydrazins oder -1,2-disulfohydrazins und insbesondere Phenylhydrazin verwendet.

11. Die gemäß dem Verfahren des Patentanspruches 1 erhaltenen Verbindungen der Formel I.

**Claims**

1. A process for producing a compound of the formula I

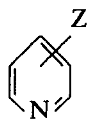

(I)

wherein

Z is —CHO or 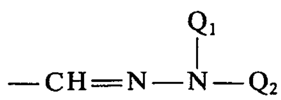

$Q_1$ is hydrogen or alkyl having 1–4 C atoms, and
$Q_2$ is phenyl which can be substituted by halogen atoms, alkyl or alkoxy groups each having 1–4 C atoms, or is naphthyl,

which process comprises reacting an aminomethylpyridine with at least a stoichiometric amount of a compound of the formula II

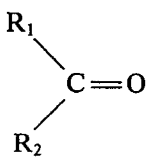

(II)

to give a compound of the formula III

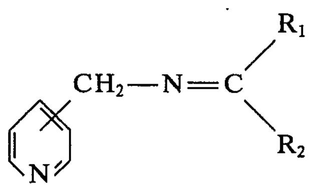

(III)

isomerising the compound of the formula III in the presence of a catalyst of the formula IV

$$(X)^{n\oplus}(OY)_n^{\ominus} \qquad (IV)$$

in an inert organic solvent, to obtain a compound of the formula V

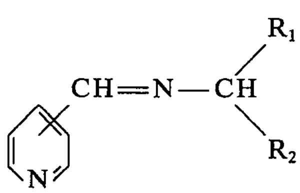

(V)

where in the formulae II to V

$R_1$ is hydrogen or alkyl having 1–8 C atoms,
$R_2$ alkyl having 1–8 C atoms, or
$R_1$ and $R_2$ together are alkylene having 4–8 C atoms,
X is an alkali metal ion or an alkaline-earth metal ion, or the ammonium group

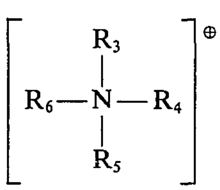

wherein $R_3$ to $R_6$ independently of one another are each straight-chain or branched-chain

alkyl ($C_1-C_{20}$), or alkyl ($C_1-C_{20}$) substituted by phenyl or naphthyl,

Y is hydrogen or alkyl having 1–12 C atoms, and

n is the charge of the alkali metal ion or alkaline-earth metal ion,

and converting the compound of the formula V into a compound of the formula I wherein Z is $-CHO$ by treating the compound of the formula V with an inorganic acid, or converting the compound of the formula V into a compound of the formula I wherein Z is the

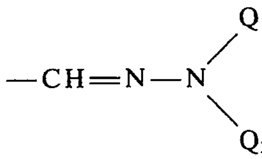 group

by reacting the compound of the formula V, in the presence of an acid, with a compound of the formula VI

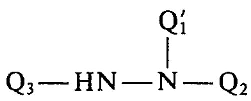 (VI)

wherein

$Q_2$ has the meanings defined under the formula I,
$Q_1'$ is hydrogen, alkyl having 1–4 C atoms or $-SO_3^{\ominus}M^{\oplus}$,
$Q_3$ is hydrogen or $-SO_3^{\ominus}M^{\oplus}$, and
$M^{\oplus}$ is an alkali metal cation.

2. A process according to Claim 1, wherein the reaction of the aminomethylpyridine with a compound of the formula II is performed in the presence of an inert organic solvent.

3. A process according to Claim 1, wherein the compounds of the formula I are produced using aminomethylpyridine as a starting material in the single-vessel process.

4. A process according to Claim 1, wherein isomerisation of the compound III is performed at a temperature of between 0 and 80° C, particularly between 10 and 60° C.

5. A process according to Claim 1, wherein there are used 3-aminomethylpyridine and particularly 4-aminomethylpyridine.

6. A process according to Claim 1, wherein there is used a compound of the formula II in which $R_1$ and $R_2$ are each ethyl or together they are pentamethylene, or in which $R_1$ is hydrogen and $R_2$ is isopropyl.

7. A process according to Claim 1, wherein there is used a compound of the formula IV in which X is an alkali metal ion, especially the sodium or potassium ion, and Y is alkyl having 1–4 C atoms, particularly tert-butyl.

8. A process according to Claim 1 for producing a compound of the formula I wherein Z is

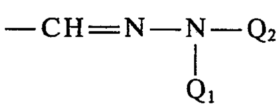

and $Q_1$ and $Q_2$ have the meanings defined under the formula I, in which process the reaction of a compound of the formula V with a compound of the formula VI in which $Q_3$ is hydrogen and $Q_1'$ is hydrogen or alkyl having 1–4 C atoms is performed in the presence of an inert organic solvent and of an organic acid, and the reaction with a compound of the formula VI in which $Q_3$ and/or $Q_1'$ are (or is) an $-SO_3^{\ominus}M^{\oplus}$ group is performed in an aqueous medium and in the presence of an inorganic acid.

9. A process according to Claim 8, wherein the organic acid used is an aliphatic or aromatic monocarboxylic acid or monosulfonic acid.

10. A process according to Claim 8, wherein the compound of the formula VI used is N-phenyl-N-methylhydrazine, the sodium salt of 1-(4'-methoxyphenyl)-2-sulfohydrazine or -1,2-disulfohydrazine, or in particular phenylhydrazine.

11. The compounds of the formula I obtained by the process of Claim 1.

## Revendications

1. Procédé de préparation d'un composé de formule I:

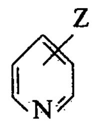

(I)

où

Z représente $-CHO$ ou $-CH=N-N(Q_1)-Q_2$

$Q_1$ représente l'hydrogène ou un reste alkyle ayant 1 à 4 atomes de carbone, et

$Q_2$ représente un reste phényle, qui peut être substitué par des atomes d'halogène, des groupes alkyle ou alcoxy ayant chacun 1 à 4 atomes de carbone, ou un reste naphtyle,

caractérisé par le fait que l'on fait réagir une aminométhylpyridine avec une quantité au moins stoechiométrique d'un composé de formule II:

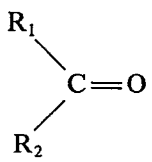

(II)

pour avoir un composé de formule III:

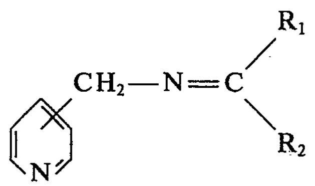

(III)

que l'on isomérise le composé de formule III en présence d'un catalyseur de formule IV:

$$(X)^{n\oplus}(OY)_n^{\ominus} \qquad \text{(IV)}$$

dans un solvant organique inerte pour avoir un composé de formule V:

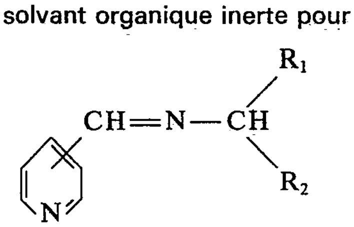

(V)

où, dans les formules II à V,

$R_1$ représente l'hydrogène ou un reste alkyle ayant 1 à 8 atomes de carbone,

$R_2$ représente un reste alkyle ayant 1 à 8 atomes de carbone, ou bien

$R_1$ et $R_2$ représentent ensemble un reste alkylène ayant 4 à 8 atomes de carbone,

X représente un ion de métal alcalin ou un ion de métal alcalino-terreux, ou le groupe ammonium

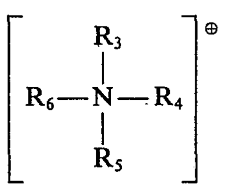

où $R_3$ à $R_6$ représentent indépendamment les uns des autres un reste alkyle $(C_1-C_{20})$ non substitué, ramifié ou non ramifié ou bien un reste alkyle $(C_1-C_{20})$ substitué par un reste phényle ou naphtyle,

Y représente l'hydrogène ou un reste alkyle ayant 1 à 12 atomes de carbone, et

n représente la charge de l'ion de métal alcalin ou de l'ion de métal alcalino-terreux,

et que l'on transforme le composé de formule V en un composé de formule I, où Z représente $-CHO$, en traitant le composé de formule V avec un acide minéral, ou bien que l'on transforme le composé de formule V en un composé de formule I, où Z représente le groupe:

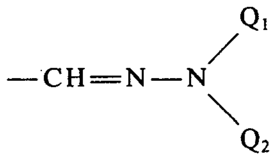

en faisant réagir le composé de formule V, en présence d'un acide, avec un composé de formule VI:

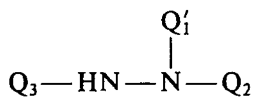

(VI)

où

$Q_2$ a la signification indiquée sous la formule I,

$Q_1'$ est l'hydrogène, un reste alkyle ayant 1 à 4 atomes de carbone, ou $-SO_3^{\ominus}M^{\oplus}$,

$Q_3$ est l'hydrogène ou $-SO_3^{\ominus}M^{\oplus}$ et

$M^{\oplus}$ représente un cation de métal alcalin.

2. Procédé selon la revendication 1, caractérisé par le fait que l'on effectue la réaction de l'aminométhylpyridine avec un composé de formule II en présence d'un solvant organique inerte.

3. Procédé selon la revendication 1, caractérisé par le fait que l'on prépare les composés de formule I à partir d'aminométhylpyridine dans le procédé en une opération.

4. Procédé selon la revendication 1, caractérisé par le fait que l'on effectue l'isomérisation du composé III à une température comprise entre 0 et 80° C et en particulier entre 10 et 60° C.

5. Procédé selon la revendication 1, caractérisé par l'utilisation de la 3-aminométhylpyridine et en particulier de la 4-aminométhylpyridine.

6. Procédé selon la revendication 1, caractérisé par le fait que l'on utilise un composé de formule II, où $R_1$ et $R_2$ représentent chacun un reste éthyle, ou bien ensemble un reste pentaméthylène, ou bien où $R_1$ représente l'hydrogène et $R_2$ un reste isopropyle.

7. Procédé selon la revendication 1, caractérisé par le fait que l'on utilise un composé de formule IV, où X représente un ion de métal alcalin, en particulier l'ion sodium ou l'ion potassium, et Y représente un reste alkyle ayant 1 à 4 atomes de carbone, en particulier tert-butyle.

8. Procédé selon la revendication 1 pour préparer un composé de formule I, où Z représente

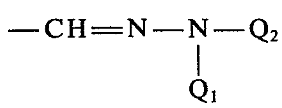

et $Q_1$ et $Q_2$ ont la signification indiquée sous la formule I, caractérisé par le fait que l'on effectue la réaction d'un composé de formule V avec un composé de formule VI, où $Q_3$ représente l'hydrogène et $Q_1'$ représente l'hydrogène ou un reste alkyle ayant 1 à 4 atomes de carbone, en présence d'un solvant organique inerte et d'un acide organique, et la réaction avec un composé de formule VI, où $Q_3$ et/ou $Q_1'$ représentent un groupe $-SO_3^{\ominus}M^{\oplus}$, en milieu aqueux et en présence d'un acide minéral.

9. Procédé selon la revendication 8, caractérisé par le fait que l'on utilise comme acide organique, un mono-acide carboxylique ou un mono-acide sulfonique, aliphatiques ou aromatiques.

10. Procédé selon la revendication 8, caractérisé par le fait que l'on utilise comme composé de formule VI, la N-phényl-N-méthylhydrazine, le sel de sodium de la 1-(4'-méthoxyphényl)-2-sulfohydrazine ou -1,2-disulfohydrazine, et en particulier la phénylhydrazine.

11. Les composés de formule I obtenus conformément au procédé de la revendication 1.